# FASCICULE DE BREVET EUROPEEN

(11) **EP 2 391 283 B1**
(45) Date de publication et mention de la délivrance du brevet: **27.03.2013**
(21) Numéro de dépôt: 09803789.8
(22) Date de dépôt: 17.12.2009
(51) Int. Cl.: A61B 17/30, A61F 9/011

(54) **PINCE DE MICROCHIRURGIE, EN PARTICULIER PINCE À CAPSULO-RHÉXIS PAR MICROINCISION.**
MIKROCHIRURGISCHE PINZETTE, IM SPEZIELLEN MIKROINVASIVE CAPSULORHEXIS-PINZETTE
MICROSURGERY CLAMP, IN PARTICULAR MICROINCISION CAPSULORHEXIS CLAMP

(30) Priorité: 27.01.2009 FR 0900349
(43) Date de publication de la demande: 07.12.2011
(73) Titulaire: CDI Hong Kong Limited, Hong Kong (CN)
(72) Inventeur: Andre, Jean-Marie, 13001 Marseille (FR)
(74) Mandataire: Marek, Pierre
(86) Numéro de dépôt international: PCT/FR2009/001439
(87) Numéro de publication internationale: WO 2010/100338

(56) Documents cités:
- US-A- 5 167 618

## Description

La présente invention se rapporte au domaine chirurgical et trouve une application particulièrement intéressante dans celui de l'opération de la cataracte. Plus précisément, elle concerne une pince chirurgicale de précision qui peut être, en particulier, une pince à capsulo-rhéxis.

L'opération de la cataracte comporte une étape chirurgicale communément appelée le "capsulo-rhéxis", qui consiste en la réalisation d'une ouverture circulaire et continue dans la capsule antérieure du cristallin. Au cours de cette étape, une pince spéciale, appelée pince à capsulo-rhéxis, est introduite par une incision cornéenne ou scléro-coméenne jusqu'à la capsule antérieure du cristallin, dont elle agrippe un lambeau pour réaliser une ouverture circulaire et continue dans cette dernière. Apres son retrait, la portion déchirée laisse place à l'ouverture voulue.

Plus précisément, selon un mode d'exécution connu (par exemple US-5.167.618), une pince à capsulo-rhéxis comporte deux branches symétriques reliées l'une à l'autre par l'une de leurs extrémités et formant la partie proximale de manipulation de la pince et dont les extrémités opposées sont constituées par des pointes de pincement recourbées dans une direction perpendiculaire au plan de rapprochement desdites branches, ces pointes de pincement extrêmement fines étant ainsi susceptibles d'être rapprochées élastiquement l'une de l'autre et formant la partie distale active de ladite pince.

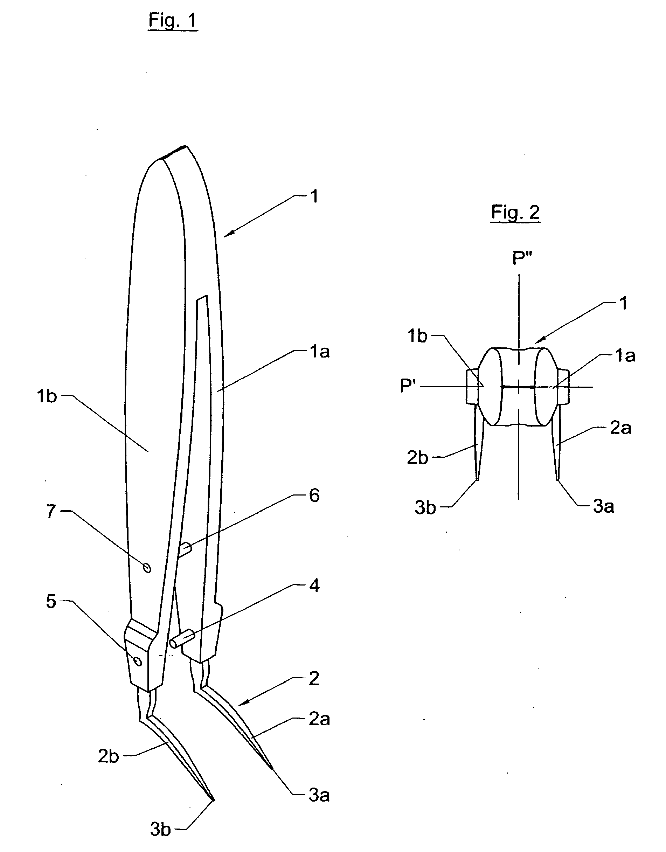

Une pince à capsulo-rhéxis de ce genre est représentée à la figure 1 des dessins annexés.

On conçoit qu'il est souhaitable que l'incision cornéenne ou scléro-cornéenne soit la plus petite possible.

Actuellement, les pinces à rhéxis du genre susmentionné peuvent fonctionner en passant au travers d'une incision cornéenne d'une longueur d'au moins 3 mm.

Une réduction de la dimension de cette incision est cependant souhaitable, notamment pour éviter une suture, favoriser la cicatrisation, et obtenir de meilleurs résultats post opératoires.

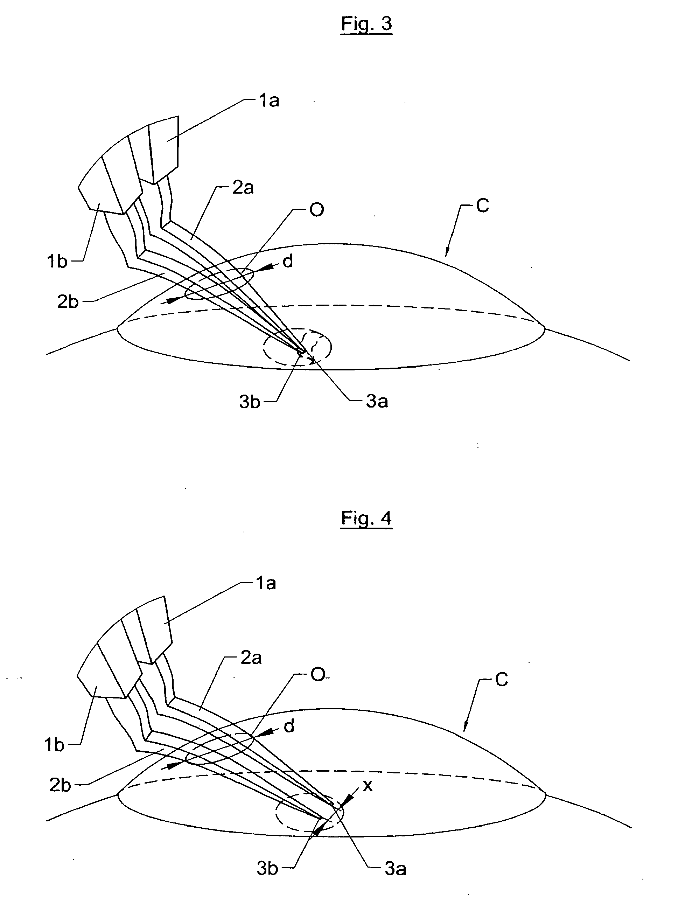

Toutefois, si une pince à rhéxis classique peut fonctionner en passant à travers une incision cornéenne de 3 mm, son fonctionnement devient difficile voire impossible en passant par une ouverture de longueur inférieure. En effet, dans cette hypothèse, les pointes de pincement après avoir été introduites en position rapprochée dans ladite incision (figure 3), n'auraient pas un espace suffisant pour pouvoir s'écarter (figure 4), afin de permettre de saisir un lambeau de la capsule antérieure puis de le relâcher après exécution de l'ouverture dans ladite capsule.

L'évolution des techniques de chirurgie tend, en général, vers une réduction de la taille des incisions d'accès aux organes à opérer, et, dans le cas de l'opération de la cataracte, une réduction de l'incision cornéenne ou scléro-cornéenne.

L'invention a notamment pour but de mettre à disposition des chirurgiens ophtalmologistes une pince à rhéxis pouvant remplir sa fonction en passant à travers une ouverture cornéenne de 1.5 mm.

Selon l'invention, ce but est atteint grâce à une pince de microchirurgie, en particulier une pince à capsulo-rhéxis par microincision, comprenant une partie proximale de manipulation ou manche, formée de deux branches ou bras reliées l'une à l'autre par l'une de leurs extrémités, de sorte à pouvoir être rapprochées ou éloignées élastiquement, et une partie distale de pincement constituée par deux lames étroites et minces de pincement reliées aux extrémités distales des branches de manipulation et orientées latéralement par rapport à ces dernières, l'extrémité distale de chacune de ces lames de pincement étant courbée dans une direction opposée à celle du manche, de sorte à constituer une pointe de pinçage, cette pince étant notamment remarquable en ce que ses lames de pincement sont asymétriques et orientées dans un plan correspondant au plan de rapprochement et d'écartement des branches de manipulation, lesdites lames de pincement étant disposées l'une au-dessus de l'autre, de sorte que le rapprochement ou l'écartement desdites branches de manipulation entraîne le rapprochement ou l'écartement, respectivement, desdites pointes de pinçage constituant les mors de la pince.

Selon un mode d'exécution avantageux, les lames de pincement sont conformées pour pouvoir glisser l'une sur l'autre lors des mouvements de rapprochement et d'écartement des branches de manipulation.

Selon un autre mode de réalisation intéressant, l'une au moins des lames de pincement est réalisée dans un matériau flexible élastiquement. De préférence, les deux branches de pincement sont réalisées dans un matériau flexible élastiquement.

Selon une autre disposition avantageuse, les lames de pincement ont une forme courbe.

Selon une autre disposition caractéristique, le rayon de courbure de la lame de pincement supérieure est plus petit que le rayon de courbure de la lame de pincement inférieure.

Suivant une autre disposition caractéristique, la lame de pincement supérieure est plus courte que la lame de pincement inférieure.

Selon une autre disposition caractéristique, la pointe de pinçage ou mors de la lame de pincement inférieure présente, dans sa partie supérieure, une largeur plus importante que celle de la portion distale de la lame de pincement supérieure, de sorte à déborder sur chaque côté de cette dernière.

Selon un mode d'exécution préféré, les pointes de pinçage ont une forme triangulaire, et leurs parties inférieures sont superposables, en position de rapprochement.

On comprend que dans l'étape opératoire d'exécution du capsulo-rhéxis, la pince de microchirurgie selon l'invention peut remplir sa fonction en passant à travers une incision cornéenne de très petite dimension, inférieure à 2 mm, par exemple à travers une incision de seulement 1.5 mm.

En effet, la largeur de la partie invasive de la pince, constituée par les portions recourbées superposée des lames de pincement, ne varie pas durant les manipulations de la pince, que celle-ci soit ouverte ou fermée.

En outre, malgré sa grande précision, cette pince de microchirurgie peut être fabriquée industriellement sous forme d'un article jetable, à usage unique.

Les buts, caractéristiques et avantages ci-dessus, et d'autres encore, ressortiront mieux de la description qui suit et des dessins annexés dans lesquels
La figure 1 est une vue en perspective d'une pince à rhéxis classique.
La figure 2 est une vue de dessus de cette pince.
Les figures 3 et 4 sont des vues de détail, en perspective et à plus grande échelle, illustrant le fonctionnement d'une pince à rhéxis classique.

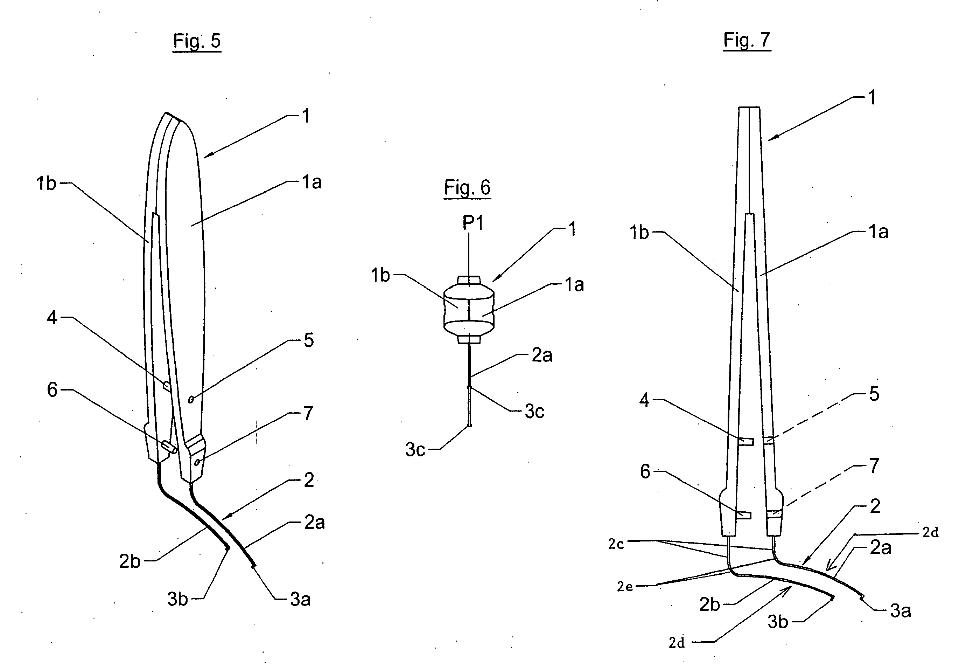

La figure 5 est une vue en perspective d'une pince à rhéxis selon l'invention, représentée en position ouverte.
La figure 6 est une vue de dessus de cette pince.
La figure 7 est une vue de côté de cette dernière.

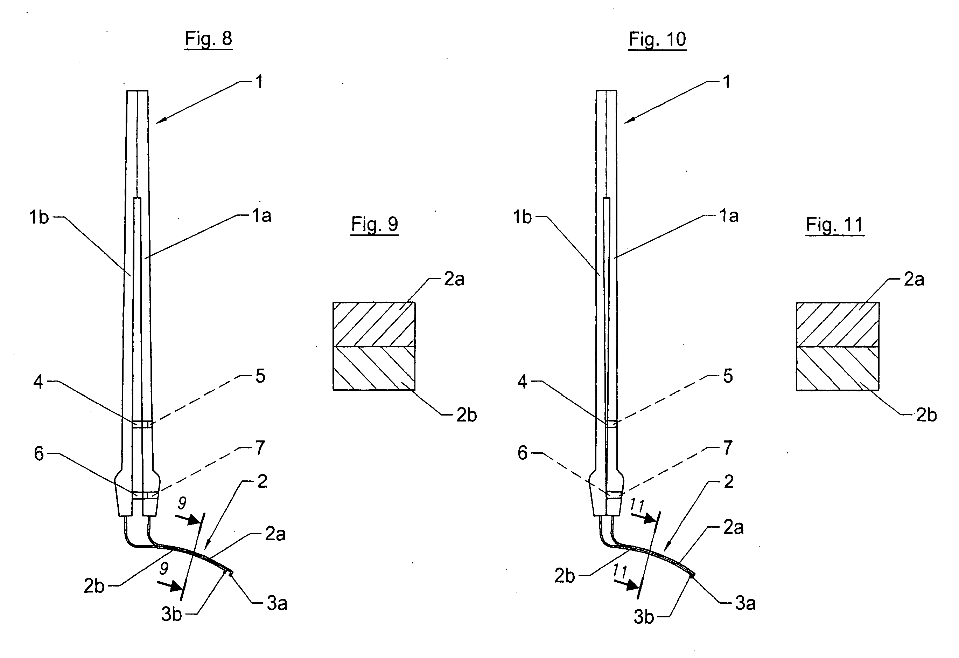

La figure 8 est une vue de côté montrant le rapprochement des branches de manipulation et des pointes de pinçage de la pince, dans une position permettant de saisir la capsule antérieure du cristallin.
La figure 9 est une vue en coupe et à plus grande échelle, selon la ligne 9-9 de la figure 8.
La figure 10 est une vue de côté représentant la pince en position de complète fermeture.
La figure 11 est une vue en coupe et à plus grande échelle, selon la ligne 11-11 de la figure 10.

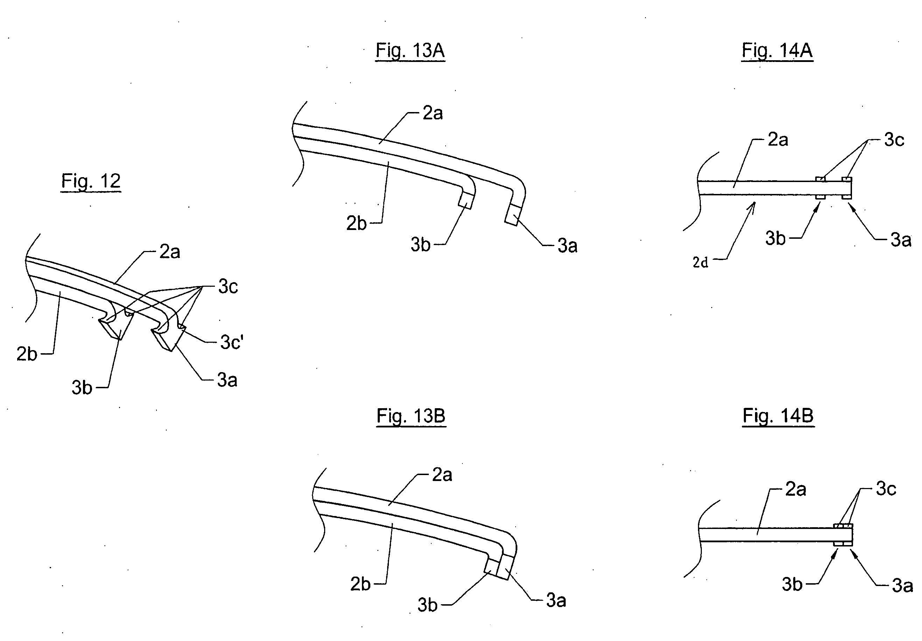

La figure 12 est une vue en perspective et à plus grande échelle, des extrémités distales des lames de pincement et de leurs pointes de pinçage.
La figure 13A est une vue de détail, de côté et à plus grande échelle, montrant les extrémités distales des branches de pincement de la pince, représentées dans la position de rapprochement initial montrée sur la figure 8.
La figure 13B est une vue analogue à la figure 13A et montrant la pince dans la position de complète fermeture représentée sur la figure 10.
Les figures 14A et 14B sont de vues de dessus des figures 13A et 13B, respectivement.

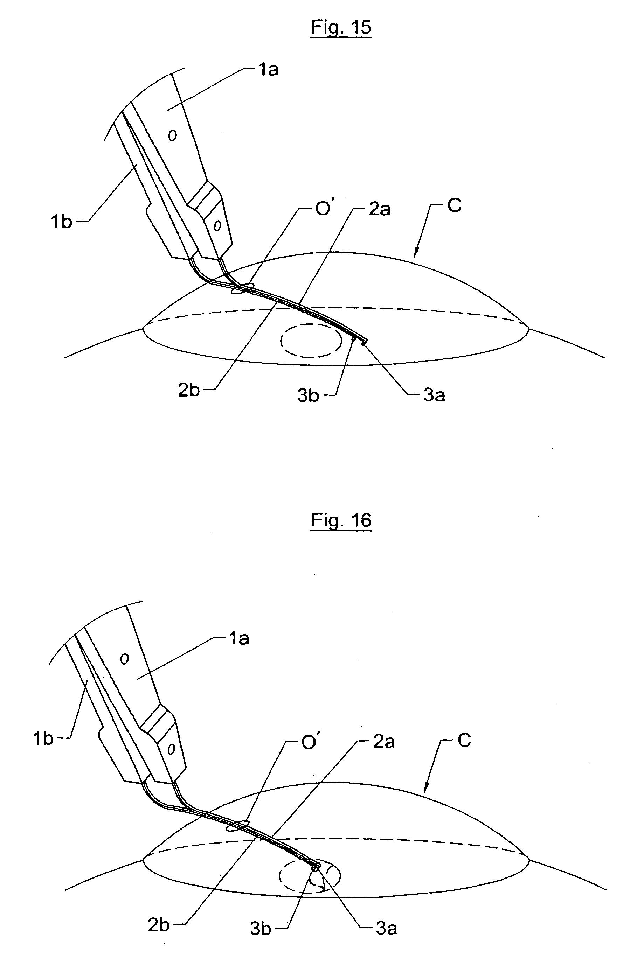

Les figures 15 et 16 sont de vues en perspective illustrant le fonctionnement d'une pince à rhéxis selon l'invention, en intra-oculaire.

On se reporte auxdits dessins pour décrire un exemple de réalisation avantageux, quoique nullement limitatif, de la pince de microchirurgie selon l'invention.

D'autre part, on décrit ci-après une application particulièrement intéressante de l'invention à la réalisation d'une pince à rhéxis, mais on souligne encore que l'invention peut, bien entendu, être mise en oeuvre pour la fabrication de pinces de microchirurgie plus spécialement adaptées à l'exécution d'autres opérations chirurgicales susceptibles d'être effectuées à travers une incision de très petite dimension.

On souligne que pour faciliter la compréhension de l'invention, on utilise, dans la description et dans les revendications, des termes tels que "supérieure", "inférieure", "dessus", "dessous", "haute", "basse", par référence à la position verticale ou proche de la verticale du manche de la pince microchirurgicale lors de l'exécution d'un rhéxis ; ces termes n'ont donc aucun caractère limitatif.

La pince à rhéxis selon l'invention est, de préférence, du genre comportant principalement : d'une part, une partie proximale de manipulation 1 ou manche, formée de deux branches ou bras 1 a, 1b, rattachées l'une à l'autre par l'une de leurs extrémités, de sorte à pouvoir être rapprochées ou éloignées élastiquement, et une partie distale de pincement 2 constituée par deux lames étroites et minces de pincement 2a, 2b reliées aux extrémités distales des branches de manipulation 1 a, 1b et orientées latéralement par rapport à ces dernières, l'extrémité distale de chacune de ces lames de pincement 2a, 2b étant courbée dans une direction opposée à celle du manche 1, de sorte à constituer une pointe de pinçage 3a, 3b.

Comme le montre la figure 2, les lames de pincement 2a, 2b, d'une telle pince classique de microchirurgie oculaire sont orientées latéralement dans une direction ou plan P" perpendiculaire à la direction ou plan P' dans laquelle s'opère la rapprochement et l'écartement des branches 1a, 1 b de ladite pince. En outre, une telle pince peut être avantageusement munie de moyens de centrage et de pré-centrage constitués par des ergots ou doigts 4, 6 solidaires de l'une des branches et orientés en direction de l'autre branche, et par des orifices 5, 7 ménagés dans l'autre branche, en regard desdits ergots ou doigts.

Une pince à rhéxis classique de ce genre est représentée à la figure 1 et son mode de fonctionnement est illustré aux figures 3 et 4.

Au départ, la pince à rhéxis est au repos et ne subit donc aucun effort de mise en pression; les lames de pincement 2a, 2b sont écartées l'une de l'autre. En exerçant une pression simultanée sur les deux branches 1 a, 1 b de la pince, on amène, dans un premier temps, les extrémités des lames de pincement 2a, 2b au contact l'une de l'autre. Lesdites lames de pincement 2a, 2b sont alors introduites dans cette position, dans le globe oculaire, à travers l'incision cornéenne O (figure 3) préalablement pratiquée dans la cornée C et dont la grandeur d est nécessairement supérieure ou égale à 3 mm. En effet, les extrémités ou parties de pinçage 3a, 3b des lames de pincement 2a, 2b doivent ensuite être écartées d'une distance X (figure 4) pour pouvoir saisir le lambeau de capsule antérieure (préalablement créé, lors d'une phase opératoire précédente). On comprend que la longueur d de l'incision O doit nécessairement, pour permettre l'écartement des lames de pincement 2a, 2b, à l'intérieur de l'oeil, être plus importante que la longueur de l'incision qui serait nécessaire pour la seule introduction desdites lames de pincement en position rapprochée, dans l'oeil. Pour que cet écartement X soit possible, il faut que l'incision ou ouverture O ait une dimension d'au moins 3 mm, comme cela vient d'être expliqué, cette dimension correspondant à la distance d comprise entre les faces externes des lames de pincement 2a, 2b, en position d'écartement de celles-ci (figure 4), au niveau de leur engagement dans ladite incision. En exerçant ensuite à nouveau une pression sur les branches 1a, 1b, ces dernières se rapprochent davantage en entraînant la mise sous tension élastique des lames de pincement flexibles 2a, 2b, la force de préhension communiquée aux extrémités de celles-ci permet alors d'agripper la capsule antérieure du cristallin et de réaliser le rhéxis.

Selon une importante disposition caractéristique de l'invention, les lames de pincement 2a, 2b de la pince sont asymétriques et orientées dans une direction ou plan P1 correspondant à la direction ou plan de rapprochement et d'écartement des branches de manipulation 1a, 1b, lesdites lames de pincement 2a, 2b étant disposées l'une au-dessus de l'autre, de sorte que le rapprochement ou l'écartement desdites branches de manipulation 1 a, 1 b entraîne le rapprochement ou l'écartement, respectivement, des pointes de pinçage 3a, 3b constituant les mors de la pince.

Selon un mode de réalisation avantageux, les lames de pincement 2a, 2b sont conformées pour pouvoir glisser l'une sur l'autre lors des mouvements de rapprochement et d'écartement des branches de manipulation 1 a, 1 b.

Selon un autre mode d'exécution avantageux, l'une au moins des lames de pincement 2a, 2b est réalisée dans un matériau flexible élastiquement.

De préférence, les deux branches de pincement 2a, 2b sont réalisées dans un matériau flexible élastique.

De manière avantageuse, la pince de microchirurgie selon l'invention peut être fabriquée industriellement sous forme d'un outil jetable, à usage unique.

Dans ce cas, les branches de manipulation 1 a, 1b constituant le manche de la pince peuvent être obtenues en matière plastique bio-compatible, à mémoire de forme, par tout procédé de moulage adéquat tandis que les lames de pincement 2a, 2b sont réalisées dans un métal bio-compatible, à mémoire de forme, par exemple en acier inoxydable.

Suivant le mode d'exécution illustré à titre d'exemple, chacune des lames de pincement 2a, 2b, comporte une partie proximale ou queue d'ancrage rectiligne 2c implantée de moulage dans l'extrémité distale d'une branche 1a ou 1b et une partie distale recourbée 2d reliée à ladite queue d'ancrage par un coude 2e en formant un angle avec celle-ci, et se terminant par une pointe de pinçage 3a ou 3b, constituant l'un des mors de la pince.

Les portions distales 2d des lames de pincement 2a, 2b sont effilées et s'amenuisent progressivement en direction de leur extrémité libre. Lesdites lames présentent, par exemple, dans cette portion, une largeur variant de 0.6 mm à 0.4 mm, et une épaisseur de 0.3 mm.

De préférence, les portions distales 2d des lames de pincement 2a, 2b ont une largeur identique, de sorte qu'en position de fermeture de la pince, lesdites parties distales sont superposables.

Avantageusement, l'une au moins des lames de pincement 2a, 2b a une forme courbe.

De préférence, les deux lames de pincement 2a, 2b, ont une forme courbe, dans leur portion orientée latéralement par rapport au manche 1.

Dans ce cas, le rayon de courbure de la lame de pincement supérieure 2a est plus petit que le rayon de courbure de la lame de pincement inférieure 2b.

On observe que la lame de pincement supérieure 2a est plus courte que la lame de pincement inférieure 2b.

Selon une autre disposition caractéristique, la pointe de pinçage 3b ou mors de la lame de pincement inférieure 2b présente, dans sa partie supérieure, une largeur plus importante que celle de la portion distale 2d de la lame de pincement supérieure 2a, de sorte à déborder sur chaque côté de cette dernière. La partie supérieure 3c de la pointe de pinçage 3b de la lame de pincement inférieure 2b présente, par exemple, une largeur de 0.5 mm. Les extrémités latérales débordantes 3c' de la pointe de pinçage inférieure 3b sont ainsi repérables par le chirurgien ophtalmologiste, en cours d'intervention, en lui permettant ainsi de vérifier le bon écartement (environ 2 mm) des pointes de pinçage 3a, 3b nécessaires pour saisir le lambeau de capsule antérieur.

Selon un mode d'exécution préféré, les pointes de pinçage 3a, 3b ont une forme triangulaire, et leurs parties inférieures sont superposables, en position de rapprochement. La pointe de pinçage supérieure 3a présente, par exemple, une hauteur de 0.4 mm, tandis que la hauteur de la pointe de pinçage inférieure 3b est de 0.2 mm.

On décrit ci-après le fonctionnement de la pince à rhéxis selon l'invention.

En position de repos, aucune pression n'est appliquée sur les branches espacées 1a, 1b de la pince, de sorte que les pointes de pinçage 3a, 3b sont également espacées.

Le rapprochement des branches 1a, et 1b sous une faible pression des doigts du chirurgien permet de mettre en contact les lames de pincement 2a, 2b et les pointes de pinçage 3a, 3b.

Le rapprochement des branches 1a, 1b, de la pince entraîne l'application, l'une contre l'autre, des parties distales recourbées 2d des lames de pincement supérieure 2a et inférieure 2b qui glissement l'une sur l'autre.

Grâce à la différence de courbure initiale de ces deux parties 2d réalisées en métal très élastique (effet ressort important) la face inférieure de la lame de pincement supérieure 2a va parfaitement épouser la forme de la face supérieure de la lame de pincement inférieure 2b. C'est dans cette situation que la partie invasive de la pince à rhéxis est introduite dans l'oeil à travers une ouverture O' de 1.5 mm seulement.

Durant toutes les manipulations qui vont suivre, la largeur de la partie invasive va rester la même (entre 06 et 0.4 mm), ce qui rend possible son utilisation à travers cette très petite incision O'.

Pour pouvoir attraper et relâcher la capsule antérieure du cristallin, les mors 3a, 3b de la pince doivent pouvoir d'écarter l'un de l'autre sur une distance de l'ordre de deux millimètres.

Lors de ce mouvement « ouverture / fermeture » de deux mm, il ne subsiste aucun espace entre les deux parties distales recourbées 2d des lames de pincement supérieure 2a et inférieure 2b sur une longueur minimale de 10mm, à partir de l'extrémité distale de la lame de pincement 2a donc du mors 3a de ladite lame de pincement supérieure (10 mm « utile » intraoculaire lors du mouvement « ouverture / fermeture »).

La partie invasive 2d-2d de la pince située dans l'incision présentera donc exactement la même largeur que la pince soit fermée ou ouverte de deux mm (ouverture nécessaire pour attraper la capsule).

Cette largeur invariable est compatible avec une incision de 1.5 mm.

En relâchant quelque peu la pression sur les branches 1a, 1 b de la pince, on obtient automatiquement un écartement des pointes de pinçage 3a, 3b, sur une distance de l'ordre de deux millimètres suffisantes pour permettre la préhension de la capsule antérieure du cristallin. Cet écartement de deux millimètres des pointes de pinçage 3a, 3b est suffisant pour permettre au chirurgien de placer aisément celles-ci de part et d'autre du lambeau capsulaire et de le saisir ensuite en rapprochant de nouveau lesdites pointes de pinçage 3a, 3b. Dans cette situation, la force de pincement qui est exercée par les pointes de pinçage 3a, 3b des lames de pincement 2a, 2b, est suffisante pour saisir et manipuler la capsule antérieure du cristallin.

## Revendications

1. Pince de microchirurgie, en particulier pince à capsulo-rhexis par microincision, comprenant une partie proximale de manipulation (1) ou manche formée de deux branches ou bras (1a, 1b) rattachées l'une à l'autre par l'une de leurs extrémités, de sorte à pouvoir être rapprochées ou éloignées élastiquement, et une partie distale de pincement (2) constituée par deux lames étroites et minces de pincement (2a, 2b) reliées, respectivement, aux extrémités distales des branches de manipulation (1a, 1b) et orientées latéralement par rapport à ces dernières, l'extrémité distale de chacune de ces lames de pincement (2a, 2b) étant courbée dans une direction opposée à celle du manche (1), de sorte à constituer une pointe de pinçage (3a, 3b), **caractérisée en ce que** les lames de pincement (2a, 2b) sont asymétriques et orientées dans une direction ou plan (P1) correspondant à la direction ou plan de rapprochement et d'écartement des branches de manipulation (1a, 1b), lesdites lames de pincement (2a, 2b) étant disposées l'une au-dessus de l'autre, en considérant une position verticale dudit manche (1), de sorte que le rapprochement ou l'écartement desdites branches de manipulation (1a, 1b) entraîne le rapprochement ou l'écartement, respectivement, desdites pointes de pinçage (3a, 3b) constituant les mors de la pince.

2. Pince de microchirurgie, en particulier pince à capsulo-rhexis par microincision, selon la revendication 1, **caractérisée en ce que** les lames de pincement (2a, 2b) sont conformées pour pouvoir glisser l'une sur l'autre lors des mouvements de rapprochement et d'écartement des branches de manipulation (1a, 1b).

3. Pince de microchirurgie, en particulier pince à capsulo-rhexis par microincision, selon l'une des revendications 1 ou 2, **caractérisée en ce que** l'une au moins des lames de pincement (2a, 2b) est réalisée dans un matériau flexible élastiquement.

4. Pince de microchirurgie, en particulier pince à capsulo-rhexis par microincision, selon la revendication 3, **caractérisée en ce que** les deux branches de pincement (2a, 2b) sont réalisées dans un matériau flexible élastique.

5. Pince de microchirurgie, en particulier pince à capsulo-rhexis par microincision, selon l'une quelconque des revendications 1 à 4, **caractérisée en ce que** l'une au moins des lames de pincement (2a, 2b) a une forme courbe.

6. Pince de microchirurgie, en particulier pince à capsulo-rhexis par microincision, selon la revendication 5, **caractérisée en ce que** les deux lames de pincement (2a, 2b) ont une forme courbe.

7. Pince de microchirurgie, en particulier pince à capsulo-rhexis par microincision, selon la revendication 6, **caractérisée en ce que** le rayon de courbure de la lame de pincement supérieure (2a) est plus petit que le rayon de courbure de la lame de pincement inférieure (2b).

8. Pince de microchirurgie, en particulier pince à capsulo-rhexis par microincision, selon l'une quelconque des revendications 1 à 7, **caractérisée en ce que** la lame de pincement supérieure (2a) est plus courte que la lame de pincement inférieure (2b), dans la partie distale recourbée (2d) de celles-ci.

9. Pince de microchirurgie, en particulier pince à capsulo-rhexis par microincision, selon l'une quelconque des revendications 1 à 8, **caractérisée en ce que** la pointe de pinçage ou mors (3b) de la lame de pincement inférieure (2b) présente, dans sa partie supérieure, une largeur plus importante que celle de la portion distale (2d) de la lame de pincement supérieure (2a), de sorte à déborder sur chaque côté de cette dernière.

10. Pince de microchirurgie, en particulier pince à capsulo-rhexis par microincision, selon la revendication 9, **caractérisée en ce que** les pointes de pinçage (3a, 3b) ont une forme triangulaire, et leurs parties inférieures sont superposables, en position de contact.

## Claims

1. Microsurgery clamp, particularly a microincision capsulorhexis clamp, comprising a proximal handling portion (1) or handle including two branches or arms (1a, 1b) interconnected at one of the ends thereof, so as to be suitable for being approached or separated elastically, and a distal clamping portion (2) consisting of two narrow and thin clamping blades (2a, 2b) connected to the distal ends of the handling branches (1a, 1b) and oriented laterally relative thereto, the distal end of each of the clamping blades (2a, 2b) being curved in a direction opposite to that of the handle (1), so as to form a clamping tip (3a, 3b), **characterised in that** the clamping blades (2a, 2b) are asymmetrical and oriented in a direction or plane (P1) corresponding to the approaching or separation directions of the handling branches (1a, 1b), said clamping blades (2a, 2b) being arranged above each other, relative to a vertical position of said handle (1), so that the approaching or separation movement of said handling branches (1a, 1b) results in the approaching or separation movement of said clamping tips (3a, 3b) forming the jaws of the clamp, respectively.

2. Microsurgery clamp, particularly a microincision capsulorhexis clamp, according to claim 1, **characterised in that** the clamping blades (2a, 2b) are designed to be able to slide over each other during handling branch (1a, 1b) approaching and separation movements.

3. Microsurgery clamp, particularly a microincision capsulorhexis clamp, according to any of claims 1 or 2, **characterised in that** at least one of the clamping blades (2a, 2b) is made of an elastically flexible material.

4. Microsurgery clamp, particularly a microincision capsulorhexis clamp, according to claim 3, **characterised in that** both clamping blades (2a, 2b) are made of an elastic flexible material.

5. Microsurgery clamp, particularly a microincision capsulorhexis clamp, according to any of claims 1 to 4, **characterised in that** at least one of the clamping blades (2a, 2b) has a curved shape.

6. Microsurgery clamp, particularly a microincision capsulorhexis clamp, according to claim 5, **characterised in that** both clamping blades (2a, 2b) have a curved shape.

7. Microsurgery clamp, particularly a microincision capsulorhexis clamp, according to claim 6, **characterised in that** the radius of curvature of the upper clamping blade (2a) is smaller than the radius of curvature of the lower clamping blade (2b).

8. Microsurgery clamp, particularly a microincision capsulorhexis clamp, according to any of claims 1 to 7, **characterised in that** the upper clamping blade (2a) is shorter than the lower clamping blade (2b), in the curved distal portion (2d) thereof.

9. Microsurgery clamp, particularly a microincision capsulorhexis clamp, according to any of claims 1 to 8, **characterised in that** the clamping tip or jaw (3b) of the lower clamping blade (2b) has, in the upper portion thereof, a greater width than that of the distal portion (2d) of the upper clamping blade (2a), so as to protrude on either side thereof.

10. Microsurgery clamp, particularly a microincision capsulorhexis clamp, according to claim 9, **characterised in that** the clamping tips (3a, 3b) have a triangular shape and the lower portions thereof are suitable for stacking, in the contact position.

## Patentansprüche

1. Mikrochirurgische Pinzette, im speziellen mikroinvasive Capsulorhexis-Pinzette mit einem proximalen Handhabungsabschnitt (1) oder einem Griff aus zwei Handhabungsarmen (1a, 1 b), die an einem der Enden miteinander verbunden sind, so dass sie elastisch zusammengebracht oder voneinander getrennt werden können, und einen distalen Klemmabschnitt (2), bestehend aus zwei schmalen und dünnen Klemmklingen (2a, 2b), die mit den distalen Enden der Handhabungsarme (1 a, 1 b) verbunden und seitlich dazu orientiert sind, wobei das distale Ende jedes der Klemmklingen (2a, 2b) in einer Richtung entgegengesetzt zu derjenigen des Griffes (1) gekrümmt ist, um so eine Klemmspitze (3a, 3b) auszubilden, **dadurch gekennzeichnet, daß** die Klemmklingen (2a, 2b) asymmetrisch und in einer Richtung oder Ebene (P1) entsprechend der Zusammenführungs- oder Trennungsebene der Handhabungsarme (1 a, 1 b) orientiert sind, wobei die Klemmklingen (2a, 2b) übereinander angeordnet sind, relativ zu einer vertikalen Position des Griffs (1), so dass das Zusammenführen oder Trennen der Handhabungsarme (1 a, 1 b) das Zusammenführen oder Trennen respektive der Klemmspitzen (3a, 3b) bewirkt, welche die Klemmbacken der Pinzette ausbilden.

2. Mikrochirurgische Pinzette, im speziellen mikroinvasive Capsulorhexis-Pinzette nach Anspruch 1, **dadurch gekennzeichnet, daß** die Klemmklingen (2a, 2b) derart ausgebildet sind, dass sie während des Zusammenführens oder Trennens der Handhabungsarme (1a, 1 b) übereinander gleiten können.

3. Mikrochirurgische Pinzette, im speziellen mikroinvasive Capsulorhexis-Pinzette nach einem der Ansprüche 1 oder 2, **dadurch gekennzeichnet, dass** mindestens eine der Klemmklingen (2a, 2b) aus einem elastisch flexiblen Material hergestellt ist.

4. Mikrochirurgische Pinzette, im speziellen mikroinvasive Capsulorhexis-Pinzette gemäß Anspruch 3, **dadurch gekennzeichnet, daß** die beiden Klemmklingen (2a, 2b) aus einem elastisch flexiblen Material hergestellt sind.

5. Mikrochirurgische Pinzette, im speziellen mikroinvasive Capsulorhexis-Pinzette nach einem der Ansprüche 1 bis 4, **dadurch gekennzeichnet, daß** mindestens einer der Klemmklingen (2a, 2b) eine gekrümmte Form aufweist.

6. Mikrochirurgische Pinzette, im speziellen mikroinvasive Capsulorhexis-Pinzette gemäß Anspruch 5, **dadurch gekennzeichnet, daß** die beiden Klemmklingen (2a, 2b) eine gekrümmte Form aufweisen.

7. Mikrochirurgische Pinzette, im speziellen mikroinvasive Capsulorhexis-Pinzette nach Anspruch 6, **dadurch gekennzeichnet, daß** der Krümmungsradius der oberen Klemmklinge (2a) kleiner ist als der Krümmungsradius des unteren Klemmklinge (2b).

8. Mikrochirurgische Pinzette, im speziellen mikroinvasive Capsulorhexis-Pinzette nach einem der Ansprüche 1 bis 7, **dadurch gekennzeichnet, daß** die obere Klemmklinge (2a) in dem gekrümmten distalen Abschnitt (2d) kürzer ist als die untere Klemmklinge (2b).

9. Mikrochirurgische Pinzette, im speziellen mikroinvasive Capsulorhexis-Pinzette nach einem der Ansprüche 1 bis 8, **dadurch gekennzeichnet, dass** die Klemmspitze oder Klemmbacke (3b) der unteren Klemmklinge (2b), in deren oberen Teil, eine größere Breite aufweist als die im distalen Abschnitt (2d) der oberen Klemmklinge (2a), um so auf jeder Seite hervorzustehen.

10. Mikrochirurgische Pinzette, im speziellen mikroinvasive Capsulorhexis-Pinzette nach Anspruch 9, **dadurch gekennzeichnet, daß** die Klemmspitzen (3a, 3b) eine dreieckige Form haben und ihre unteren Abschnitte in der Kontaktposition überlagert sind.
